# EUROPEAN PATENT SPECIFICATION

(11) **EP 2 077 783 B1**
(45) Date of publication and mention of the grant of the patent: **25.06.2014**
(21) Application number: 07854042.4
(22) Date of filing: 15.10.2007
(51) Int. Cl.: A61B 17/70

(54) **CENTRAL ROD CONNECTOR AND T-ROD**
ZENTRALER STANGENVERBINDER UND T-STANGE
RACCORD À TIGE CENTRALE ET TIGE EN T

(30) Priority: 17.10.2006 US 581831
(43) Date of publication of application: 15.07.2009
(73) Proprietor: Warsaw Orthopedic, Inc., Warsaw, Indiana 46581 (US)
(72) Inventor: MALANDAIN, Hugues, Mountain View, California 94043 (US); BRUMFIELD, David L., Collierville, Tennessee 38017 (US)
(74) Representative: Hutchinson, Glenn Stanley
(86) International application number: PCT/US2007/081373
(87) International publication number: WO 2008/048923

(56) References cited:
- US-A- 5 437 669
- US-A- 5 702 392

## Description

The present disclosure generally relates to orthopedic implants used for correction of spinal injuries or deformities, and more specifically, but not exclusively, concerns apparatuses for fixing a portion of the spine to allow correction or healing thereof.

In the realm of orthopedic surgery, it is well known to use implants to fix the position of bones. In this way, the healing of a broken bone can be promoted, and malformations or other injuries can be corrected. For example, in the field of spinal surgery, supporting elements, such as rods, plates, bars or other elongated elements, can be fixed to adjacent vertebrae for a number of reasons, including (a) correcting an abnormal curvature of the spine, including a scoliotic curvature, (b) to maintain appropriate spacing and provide support to broken or otherwise injured vertebrae, and (c) perform other therapies on the spinal column.

Such elongated or other supporting members have previously been connected, fixed or attached to one or more vertebrae or other bones or tissue by attaching devices that contact the tissue. The configuration of such attaching devices depends, at least in part, upon the placement or positioning of the support member and/or the attaching devices that may be desired by the surgeon, which in turn can depend on the orthopedic therapy or correction that is necessary or desired.

US5437669 (Yuan et al) describes a system and method to form a surgical construct using spinal rods such as those used in fusing vertebrae of the spine are attached with the bifurcated connectors to form the surgical construct.

### BRIEF DESCRIPTION OF THE DRAWINGS

FIG. 1 is a perspective view of an embodiment of a connector apparatus.
FIG. 2 is a side elevational view of the embodiment shown in FIG. 1.
FIG. 3 is a partially exploded side elevational view of the embodiment shown in

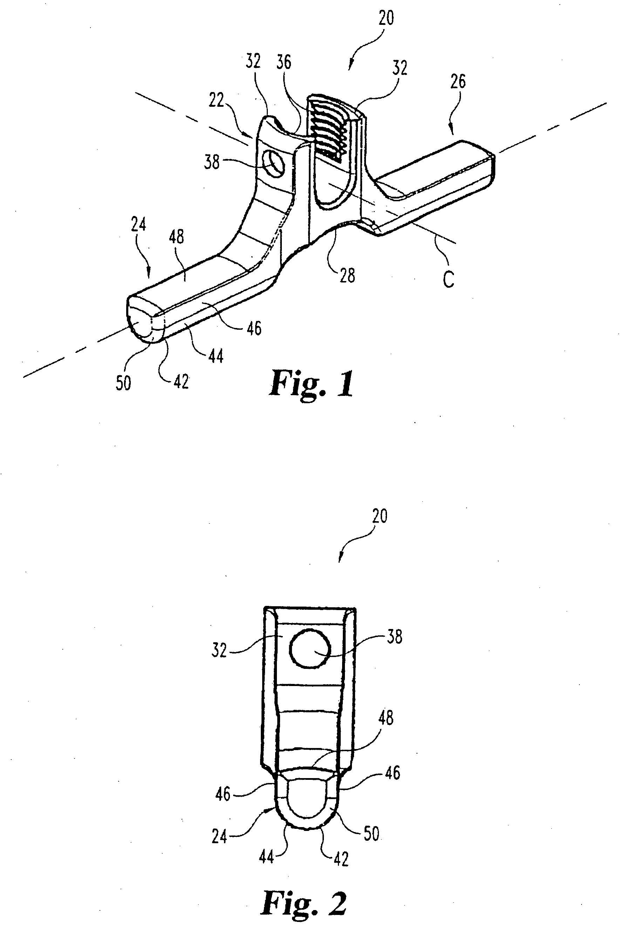

FIG. 1, rotated 90 degrees from the view shown in FIG. 2, and including additional structure.

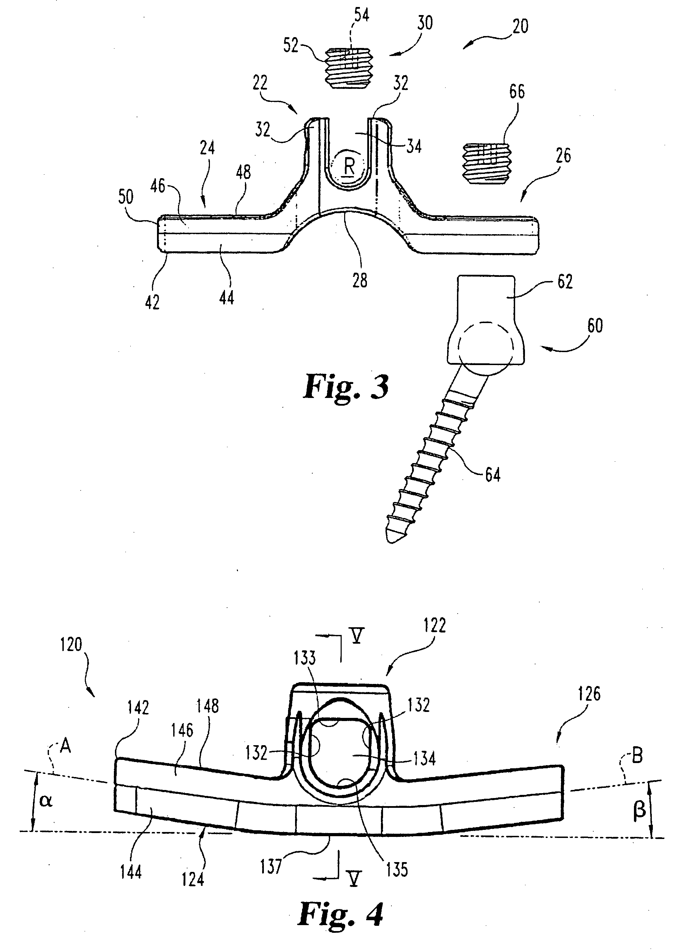

FIG. 4 is a side elevational view of an embodiment of a connector apparatus.

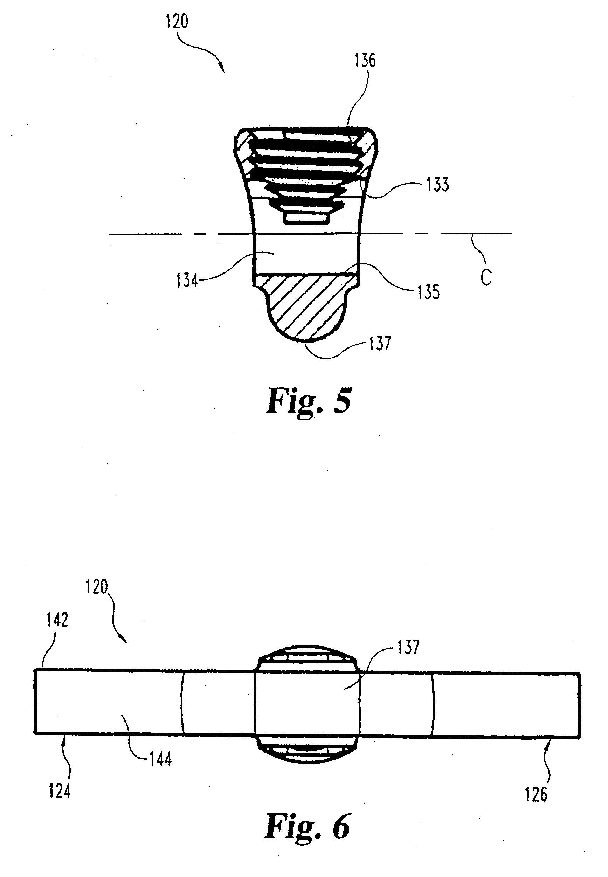

FIG. 5 is a cross-sectional view of the embodiment shown in FIG. 4, taken along the line VV in FIG. 4 and viewed in the direction of the arrows.
FIG. 6 is a bottom plan view of the embodiment shown in FIG. 4.

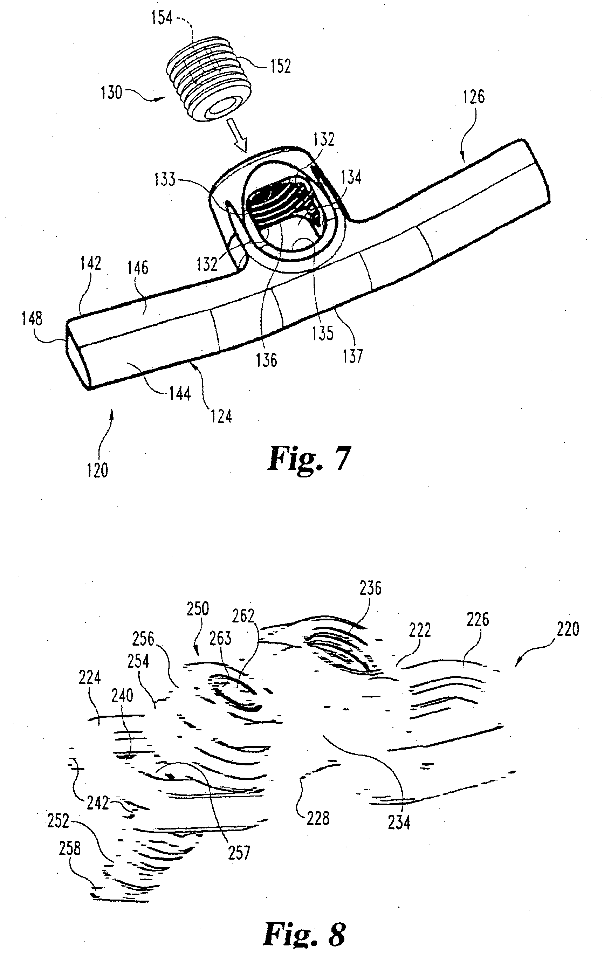

FIG. 7 is a perspective view of the embodiment shown in FIG. 4.
FIG. 8 is a perspective view of connector apparatus and additional apparatus.

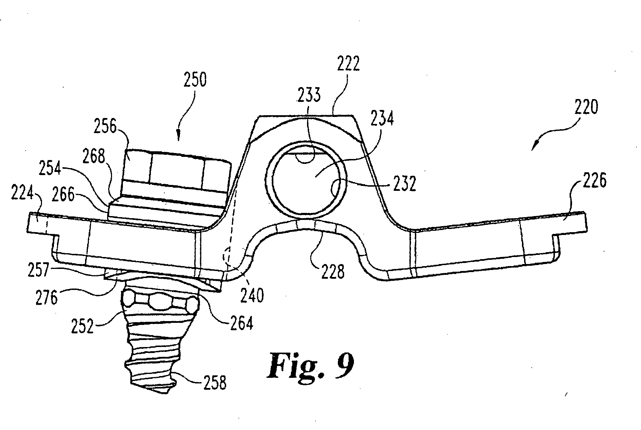

FIG. 9 is a side elevational view of the apparatuses shown in FIG. 8.

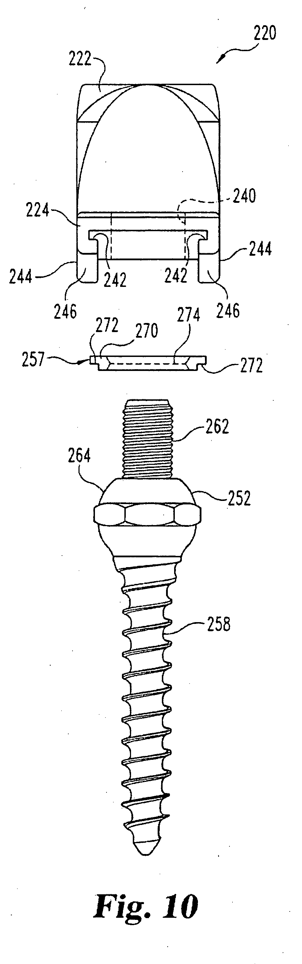

FIG. 10 is an exploded view from the side of the apparatuses shown in FIG. 8.

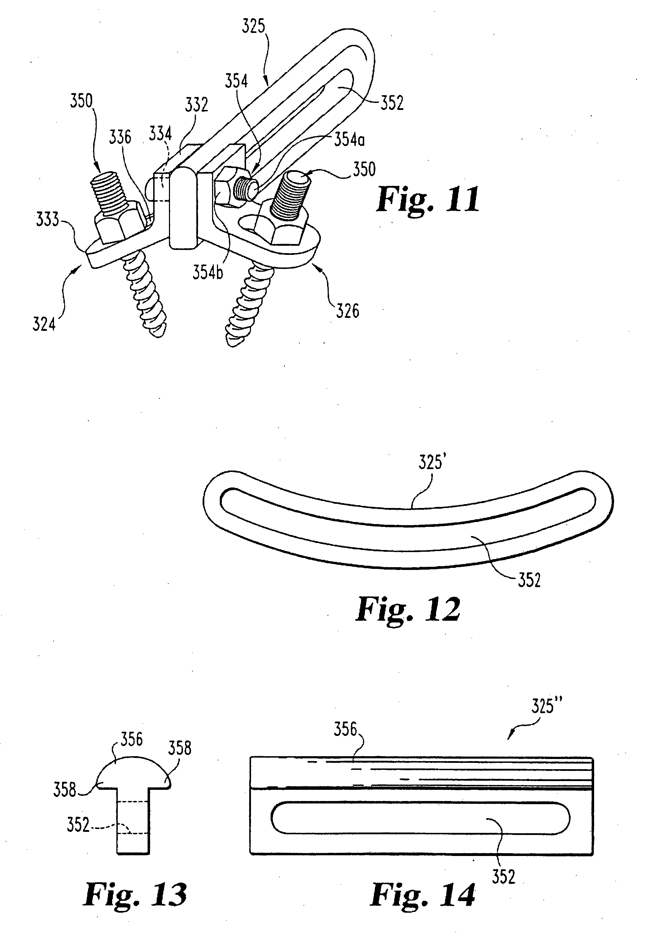

FIG. 11 is a perspective view of a connector apparatus and additional apparatus.
FIG. 12 is a side elevational view of another aspect of the apparatus shown in FIG. 11.
FIG. 13 is an end view of another aspect of the apparatus shown in FIG. 11.
FIG. 14 is a side elevational view of the apparatus shown in FIG. 13.

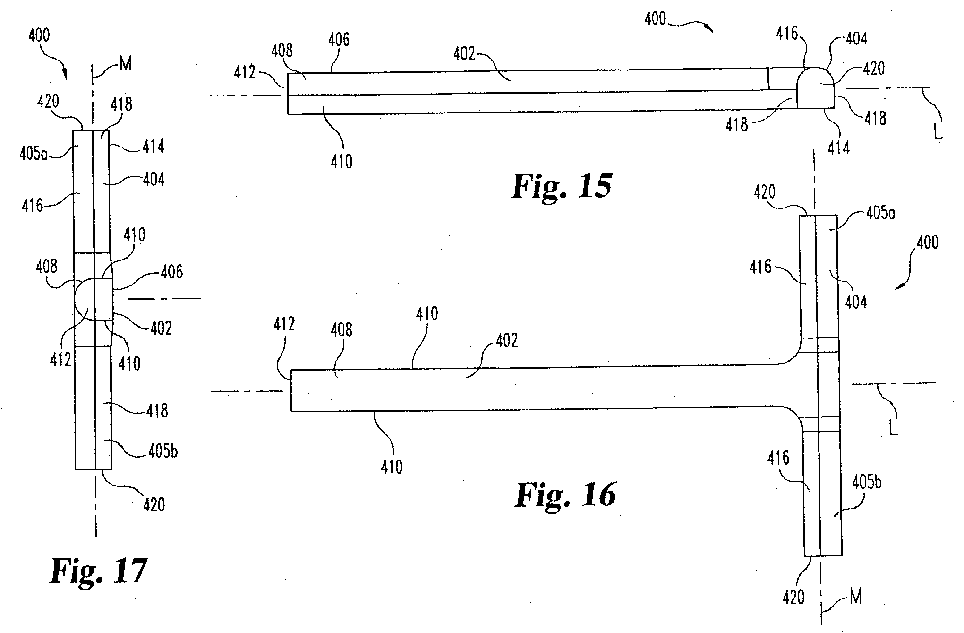

FIG. 15 is a side elevational view of an orthopedic elongated member.
FIG. 16 is a top view of the orthopedic elongated member shown in FIG. 15.
FIG. 17 is an end view of the orthopedic elongated member shown in FIG. 15.

### DESCRIPTION OF THE ILLUSTRATED EMBODIMENTS

Only the apparatuses shown in Figs. 1-7 are embodiments of the invention, whereas the apparatuses shown in Figs. 8-14 do not fall within the scope of the claims. For the purpose of promoting an understanding of the principles of the disclosure, reference will now be made to the embodiments illustrated in the drawings and specific language will be used to describe the same. It will nevertheless be understood that no limitation of the scope of the claims is thereby intended, such alterations and further modifications in the illustrated device, and such further applications of the principles of the disclosure as illustrated therein, being contemplated as would normally occur to one skilled in the art to which the disclosure relates.

Referring generally to the figures, there are shown embodiments of connecting apparatus. In one embodiment, a connecting apparatus 20 has a central portion 22, a first wing 24 and a second wing 26. In that particular embodiment, an arch 28 is formed substantially below central portion 22, and wings 24 and 26 have longitudinal axes that are substantially collinear. In other embodiments, arch 28 may be absent. Further, other embodiments may have wings 24 and 26 angled with respect to each other, so that their respective longitudinal axes are non-collinear and/or skew. Such angles may be pre-provided, e.g. apparatus 20 or individual wings 24 and/or 26 can be manufactured to have such an angle, or wings 24 and/or 26 could be bent by the surgeon just before or during a surgical procedure. A lock member 30 is also provided for connection to central portion 22.

The illustrated embodiment of central portion 22 generally has a U-shape, with two upright portions 32 defining a channel 34 extending through central portion 22. Channel 34 is shown to be substantially perpendicular to and above wings 24 and 26 in this embodiment, at least in the sense that a longitudinal axis C of channel 34 is above the collinear axis of wings 24 and 26 and would be perpendicular if the two axes intersected. Channel 34 may be otherwise oriented, for example parallel or oblique to the collinear axis of wings 24 and 26, in other embodiments. Channel axis C is generally parallel to a rod R or other elongated member situated in channel 34, which rod R is shown in the embodiment of FIG. 3 to be perpendicular to the plane of the page. In other embodiments axis C could be slanted upward or downward, i.e. non-perpendicular, to the plane of the page as viewed in FIG. 3. Branches 32 have internal threads 36 in the illustrated embodiment to accommodate lock member 30, as further described below. One or both branches may also include an indentation 38 to accommodate a projection of a gripping or holding tool (not shown), and in one embodiment indentation(s) 32 are on an exterior side of branch(es) 32.

Wings 24 and 26 are essentially identical in this embodiment, and therefore only wing 24 will be discussed for the sake of clarity. The illustrated embodiment of wing 24 is an elongated solid connected to central portion 22 and having a free end 42. In a particular embodiment wing 24 has a substantially uniform cross-section through most or all of its length. In the illustrated embodiment, wing 24 has a lower surface 44 that is substantially cylindrical, upper side surfaces 46 that are substantially planar, and a top surface 48 that is curved. The curve of top surface 48 may be a portion of a cylinder of greater radius than that of lower surface 44. The illustrated embodiment of top surface 48 shows a slight curvature, or a relatively large radius, and in other embodiments may be substantially flat or of other curvature. Free end 42 may also have curved or beveled edges 50.

Lock member 30 is illustrated in one embodiment as a set screw having external threads 52 and an internal driving print 54. Threads 52 are configured to be threadable into internal threads 36 of central portion 22. Other embodiments of set screws may be used with apparatus 20, including break-off set screws, screws with external driving prints or flats, or other threaded elements. Further, other embodiments of lock member 30 may be provided for use with apparatus 20, such as various types of clamp or other device to lock an elongated support member and apparatus 20 together.

Channel 34 of apparatus 20 is designed to accommodate at least part of an elongated member, such as a spinal rod R, and wings 24 and 26 are sized and configured to be connected to a fixation element. One embodiment of such a fixation element is bone screw 60, which includes a receiver member 62, a threaded fixation member 64, and a set screw or other lock member 66, and if receiver member 62 and fixation member 64 are movable with respect to each other, additional parts such as a crown member (not shown) for placement atop fixation member 64 may be provided. Lock member 66 may be substantially the same as lock member 30, or may take another form. A wing (e.g. wing 26) is inserted within receiver member 62 and fixation member 64 is attached to a bone, as further discussed below. One type of bone screw that can be used with apparatus 20 is the multi-axial screw shown in U.S. Patent No. 6,280,442. In that case, receiver member 62 can rotate and/or pivot with respect to fixation member 64. Other types of fixation elements that can accommodate at least a portion of wings 24 and 26, such as hooks, clamps, other types of screws, and the like, may be used with apparatus 20.

An embodiment of the use of apparatus 20 will now be described in the context of spinal corrective or therapeutic surgery. Other uses of apparatus 20 in spinal surgery or other orthopedic procedures are contemplated.

In that particular embodiment, an access to the posterior spine is obtained. The access may be an open incision, a minimally-invasive procedure, or other procedures as the surgeon may desire. Once access to the posterior spine is obtained, and the surgical site adjacent the spine is prepared (e.g. by retraction of tissue, removal of vertebral parts, or other actions), fixation elements such as bone screws 60 may be attached to one or more vertebrae. For example, where bone screws 60 are used, holes may be drilled in a vertebra to either side of a desired location for an elongated support member, and fixation members 64 may be threaded into those holes. In embodiments of apparatus 20 that include arch 28, apparatus 20 (and thus an elongated support member) can be placed at locations such that arch 28 spans a bony protrusion or other tissue, another implant, or other feature. Accordingly, with such an embodiment screws 60 may be placed on either side of such a feature. Receiver members 62 of screws 60 are oriented generally for acceptance of wings 24 and 26 of apparatus 20. Such orientation can be accomplished by rotating receiver members 62 if screw 60 is multi-axial, by rotating the entirety of screw 60 if it is fixed or uniaxial, or by other methods if other types of fixation elements are used.

When such fixation elements are in the location and orientation desired by the surgeon, apparatus 20 may be moved to the surgical site. Wing 24 is connected to one screw 60 by inserting wing 24 into a receiver member 62, in a particular embodiment, and wing 26 is connected to the other screw 60 by inserting wing 26 into another receiver member 62. Apparatus 20 may be adjusted with respect to fixation elements 60. For example, apparatus 20 can be shifted laterally, substantially parallel to the longitudinal axes of wings 24 and 26 and/or perpendicular to axis C of channel 34 of apparatus 20. As another example, apparatus 20 can be rotated substantially around the longitudinal axes of wings 24 and 26, particularly in embodiments of apparatus 20 in which some or all of wings 24 and/or 26 are cylindrical or otherwise curved. As previously noted, one or both of wings 24 and 26 may also be bent prior to or during surgery to address particular placement concerns or other needs. When apparatus 20 is situated with respect to fixation elements 60 as the surgeon desires, respective locking members 64 are connected to respective receiver members 62 to lock wings 24 and 26 and their respective receiver members 62 together.

With apparatus 20 secured to fixation elements 60, an elongated support member, such as a spinal rod R, can be inserted into channel 34 of apparatus 20. Rod R or other elongated member may be previously bent or curved, or may be curved *in situ* once placed in channel 34. Such an elongated member can be used to bridge one or more vertebral motion segments, and thus another part of rod R (not shown) may be connected to another apparatus 20, or other device, attached to another vertebra. Rod R may be turned in or moved along channel 34, and when rod R or other elongated member is in the desired position with respect to apparatus 20, lock member 30 is connected to apparatus 20. In the embodiment in which branches 32 of apparatus 20 have internal threads 36 and lock member 30 is a set screw, lock member 30 is inserted between branches 32 and threaded into threads 36 until lock member 30 contacts rod R and/or presses rod R against central portion 22 of apparatus 20. The surgeon may tighten lock member 30 to a desired extent to assure sufficient locking of apparatus 20 and rod R with respect to each other.

Adjustments to one or more aspects of the system of apparatus 20, fixation elements 60 and rod R may be made prior to final locking, as indicated above. For example, when apparatus 20 is connected to fixation elements 60, lock member(s) 66 can be loosely threaded into one or both of receiver members 62 so that apparatus 20 will not inadvertently become disconnected from receiver member(s) 62, yet apparatus 20 can be shifted, rotated or otherwise adjusted with respect to receiver member(s) 62. Similarly, when rod R is inserted into channel 34, lock member 30 may be loosely connected to apparatus 20 so as to prevent inadvertent disconnection of rod R and apparatus 20, yet allowing rod R to be bent, shifted, rotated or otherwise adjusted with respect to apparatus 20.

Prior to or after implantation of apparatus 20, as indicated in one embodiment above, other implants or surgical techniques may be used, as the surgeon may desire or deem necessary. For example, one or more additional hooks, screws, clamps, connectors or other implants may be connected to one or both of wings 24 and 26. As another example, one or more such implants may be connected to elongated member R. A second apparatus 20 could be connected to another vertebra or vertebral segment, and a portion of elongated member R may be connected to that second apparatus 20 for stability or correction of multiple vertebrae. Distraction, rotation, compression or other manipulation of vertebrae or other bones or tissue can be performed. When implantation of apparatus 20 and any associated devices is completed, and any further procedure the surgeon deems necessary is accomplished, surgical closing procedures are performed.

An embodiment of apparatus 120, similar in many ways to apparatus 20, is shown in FIGS. 4-7. Apparatus 120 has a central portion 122, a first wing 124, a second wing 126 and a lock member 130. Central portion 122 includes a channel 134 that is closed, that is, channel 134 is bounded by side walls 132 and by an upper surface 133. Channel 134 is shown to be substantially perpendicular to and above at least part of wings 124 and 126 in the illustrated embodiment. As discussed above with respect to channel 34, channel 134 may be otherwise oriented. In one particular embodiment, channel 134 has a curved lower surface 135 and upper surface 133 is substantially planar. Internal threads 136 are provided in the illustrated embodiment in upper surface 133 and/or side walls 132 to accommodate lock member 130, as further described below. A lower surface 137 is substantially horizontal in the illustrated embodiment, i.e. a longitudinal axis C of channel 134 in this embodiment, shifted downward as viewed in FIG. 4, would be substantially tangent to the lowest point of surface 137. Where surface 135b is provided, it may be substantially parallel to upper surface 133 and parallel to surface 137, as shown in FIG. 4.

Wings 124 and 126 are angled with respect to each other and with respect to surface 137 of central portion 122 in the illustrated embodiment, so that their respective longitudinal axes A and B are substantially in the same plane but non-collinear. In a particular embodiment, each of wings 124 and 126 are about seven degrees above the horizontal, or in other words the included angles between axis A and an extension of surface 137 (angle α) and between axis B and an extension of surface 137 (angle β) are each about seven degrees. In that embodiment, the included angle between axes A and B is about 166 degrees. Such a configuration of wings 124 and 126 can, in certain areas of the spine or other orthopedic surgical locations, provide a better angle or orientation of fixation elements (such as screws 60, described above) with respect to both bone and apparatus 120. As discussed above with respect to apparatus 20, longitudinal axes of wings 124 and 126 may be substantially collinear in other embodiments. Those axes may also be angled differently with respect to each other and/or to central portion 122. For example, wing 124 could be substantially horizontal, while wing 126 is angled, both wings 124 and 126 can be angled at greater or less than seven degrees, or one wing can be angled to one extent and the other angled to a lesser or greater extent.

Wings 124 and 126 are essentially identical in this embodiment, and therefore only wing 124 will be discussed for the sake of clarity. The illustrated embodiment of wing 124 is, like wing 24 discussed above, an elongated solid connected to central portion 122 and having a free end 142. In a particular embodiment wing 124 has a substantially uniform cross-section through most or all of its length. In the illustrated embodiment, wing 124 has a lower surface 144 that is substantially cylindrical and connects to surface 137 of central portion 122, upper side surfaces 146 that are substantially planar, and a top surface 148 that is substantially planar.

Lock member 130 is essentially the same as lock member 30 previously described and shown. External threads 152 are configured to be threadable into internal threads 136 of central portion 122 via application of turning force to internal driving print 154. Other embodiments of set screws or other lock members may be used with apparatus 120, as noted above.

Apparatus 120 is used, in one embodiment, in essentially the same manner as described above with respect to apparatus 20. Apparatus 120 can be used with the fixation members described above, including screws 60, other multi-axial fixation members, and the like. A variety of elongated members can be used that can be at least partially inserted into channel 134. An elongated support member that is cylindrical, or that has at least one flat side, may be used with embodiments of apparatus 120 that have a planar upper channel surface 135b or similar structure. In embodiments of apparatus 120 that are closed at the top, an elongated member must be inserted into channel 134 from the side, rather than through the top of central portion 122, as is possible with apparatus 20. Accordingly, the surgeon may connect apparatus 120 to an elongated support member, such as a spinal rod, prior to inserting the support member into the surgical site. Connection of apparatus 120 to vertebra(e) is accomplished, and the support member can then be locked with respect to apparatus 120. As discussed previously, additional implants can be implanted, and/or additional surgical procedures can be performed, prior to closing.

An embodiment of a connector apparatus 220, not according to the invention, is shown in FIGS. 8-10. Apparatus 220 includes a central portion 222 and wings 224 and 226, which in the illustrated embodiment are diametrically opposed to each other and extend from central portion 222. Wings 224 and 226 are integral with central portion 222 in this embodiment, but may also be constructed separately and assembled or attached together. In the illustrated embodiment of apparatus 220, an arch 228 is formed substantially below central portion 222. A lock member (e.g. lock member 30, described and shown above) is also provided.

Central portion 222 is similar to central portions 22 and 122 of apparatus 20 and 120 in several respects. Central portion 222, in this embodiment, includes a channel 234 that is closed, that is, channel 230 is bounded by side walls 232 and by an upper surface 233. Channel 234 is shown to be substantially perpendicular to and above at least part of wings 224 and 226 in the illustrated embodiment. As discussed above with respect to channel 34, channel 234 may be otherwise oriented. In one particular embodiment, side walls 232 are contiguous and substantially cylindrical at least in part, and extends around channel 234 in conjunction with planar upper surface 233. Internal threads 236 are provided in the illustrated embodiment in upper surface 233 and/or side walls 232 to accommodate lock member 230, as further described below.

In this embodiment, wings 224 and 226 are substantially identical, and therefore only wing 224 will be described for the sake of clarity. The illustrated embodiment of wing 224 is generally in the form of a substantially oval or rectangular flat plate and includes a generally longitudinal slot 240 through wing 224. Within slot 240 is formed a ledge 242 formed by downwardly-extending arms 244 substantially along the length of both sides of wing 224. Arms 244 have an inwardly-extending flange 246 along substantially the entire length of arms 244.

One type of a bone anchor assembly 250 for use with apparatus 220 is also shown. Generally, assembly 250 includes a bolt 252, a washer 254, and a nut 256, and may include a stabilizer 257. In the illustrated embodiment, bolt 252 includes a bone-engaging portion 258 having cancellous threads and a proximal portion 262, which is threaded with machine threads. Proximal portion 262 may include a print, such as internal hexagonal print 263, in which a driving tool (not shown) can be inserted. Between proximal portion 262 and bone-engaging portion 258, there is an intermediate portion 264 which has a rounded configuration. In one specific embodiment, the rounded shoulder has a spherical configuration.

Washer 254, in the illustrated embodiment, has a generally cylindrical body 266, a rounded or conical head 268, and an aperture therethrough. The illustrated embodiment of nut 256 is hexagonal with an internal thread configured to engage the machine threads of proximal portion 262 of bolt 252. Break-off nuts or other configurations of a nut or other locking member may be provided in other embodiments. For example, a nut having a swaged or expanded skirt or extension that extends at least part of the way through the aperture of washer 254 and holds the nut and washer together yet allows relative rotation and/or other movement between the nut and washer may be provided.

Stabilizer 257, in one embodiment, has a generally rectangular or square body 270 with an extending finger portion 272 on opposite sides of stabilizer 257. Stabilizer 257 also has a hole 274 therethrough. Fingers 272 of stabilizer 257 are shaped and dimensioned to fit within wing 224 such that fingers 272 abut ledge 242 of wing 224. In this embodiment, stabilizer 257 has a convex bottom surface 276. Stabilizer 257 may be inserted into wing 224 via its open end, and until apparatus 220 is locked with respect to bolt 252 or assembly 250 as described below, is slidable along ledge 242 of wing 224.

Apparatus 220 can be used essentially in the same manner as described above with respect to apparatus 20 and 120. Apparatus 120 can be used with the fixation members described above, including screws 60, other multi-axial fixation members, and the like. A variety of elongated members can be used that can be at least partially inserted into channel 134. An elongated support member having at least one flat side may be indicated for use with embodiments of apparatus 120 that have a planar upper channel surface 135b or similar structure. In embodiments of apparatus 120 that are closed at the top, an elongated member must be inserted into channel 134 from the side, rather than through the top of central portion 122, as is possible with apparatus 20. Accordingly, the surgeon may connect apparatus 120 to an elongated support member, such as a spinal rod, prior to inserting the support member into the surgical site. Connection of apparatus 120 to vertebra(e) is accomplished, and the support member can then be locked with respect to apparatus 120. As discussed previously, additional implants can be implanted, and/or additional surgical procedures can be performed, prior to closing.

In other embodiments, wings such as those disclosed herein need not be integral with each other or with a central portion of a connector device. Referring now generally to FIGS. 11-14, there is shown an embodiment of a connector apparatus 320, not according to the invention, having two wings 324 and 326, which are joined together on either side of an elongated support member in the form of a slotted bar or plate 325. In this embodiment, wings 324 and 326 are substantially identical, and therefore only wing 324 will be described for the sake of clarity.

Wing 324 is substantially L-shaped, having a vertical leg 332 and a lateral leg 333. Leg 332 and/or slotted bar or plate 325 may be thought of as a central portion of apparatus 320, in comparison with central portions 22, 122, 222 described above. Leg 332 includes an aperture 334 therethrough, which in one particular embodiment is a substantially circular hole. Leg 333 includes an aperture 336, which in one particular embodiment is an elongated slot. Aperture 336 may be configured substantially similarly to slot 240 discussed above, or may be a simple slot without a longitudinal channel between upper and lower surfaces for a stabilizer. Bone fixation member(s) 350, which may be similar or identical to bone fixation members 250 described above or may be otherwise configured, extend through aperture 336 and into bone. The illustrated embodiment shows one member 350 through each of wings 324 and 326, although more than one member 350 or other bone fixation member could be used in conjunction with a given wing 324 or 326, or both.

Bar or plate 325 is an elongated member having one or more slots 352, which in a particular embodiment run substantially along most of the length of bar 325. Slot 352 has a width that is comparable to a diameter or width of aperture 334 of wing 324. The overall width of bar 325 is approximately the same as or slightly larger than the height of leg 332 (i.e. the distance between leg 333 and the free end of leg 332) in a particular embodiment. A holding member 354, which in the illustrated embodiment includes a bolt 354a and a nut 354b, connect and hold together wings 324 and 326 with bar or plate 325 between them. Bar or plate 325 may be substantially rectangular, e.g. straight and planar, or may be curved as shown in bar or plate 325' in FIG. 12. Further, an embodiment of bar 325" could include an enlarged portion 356 forming flanges 358 on one or both sides of bar 325". Flange(s) 358 may be configured to contact or be adjacent to leg 332 of wing 324 (and/or the corresponding leg of wing 326) for ease of placement and adjustment.

Use of apparatus 320 may be substantially similar to the manners of use described above with respect to apparatus 20, 120 and/or 220. Once a surgical site has been appropriately prepared, wings 324 and 326 can be connected to bone (e.g. one or more vertebrae) by inserting one or more bone fixation members 350 through aperture 336 and into bone, so that each wing 324 and 326 are fixed to bone tissue. Bar 325 is inserted between wings 324 and 326, and holding member 354 is inserted through apertures 334 of each wing 324 and 326 and slot 352 of bar 325, and locked (as by locking nut 354b) to hold wings 324 and 326 to bar 325. In the illustrated embodiment, wings 324 and 326 contact bar 325 directly, and at least portions of legs of each wing 324 and 326 are substantially flush against bar 325. In other embodiments, one or both wings 324 and 326 may be separated from bar 325, with intervening space, medicaments, osteogenic substances, or other apparatus. Additionally, the combination of wings 324 and 326 with bar 325 may be accomplished prior to moving them to the surgical site, if desired. Bar 325 may be adjusted in translation and/or rotation with respect to wings 324 and/or 326 when holding member 354 is loose, and thus may have various positions with respect to wings 324 and 326. Another part of bar 325 can be connected to another set of wings 324 and 326, or to other apparatus, connected to another vertebra or set of vertebrae, so as to provide stability, therapy, or other benefit to such bones. When the surgeon is satisfied with the placement and arrangement of apparatus 320 and any apparatus to which it is connected, he or she may conclude the operation.

Many types of orthopedic procedure may be carried out along with placement of the apparatus according to one or more of the embodiments discussed above, or other embodiments. For instance, compression, distraction and/or rotation of vertebrae may be performed before, during or after placement of such apparatus. In one particular example, one apparatus 20 may be attached to one vertebra, and another apparatus 20 may be attached to a second vertebra, which may be next to the first vertebra or may have one or more vertebrae between it and the first vertebra. An elongated member is connected to each apparatus 20 as disclosed above, but is not locked to one or both apparatus 20. Compression or distraction of vertebrae can then occur, which can result in one or both apparatus 20 moving along the elongated member. While the vertebrae are held in the compressed or distracted state, final locking of the loosely connected apparatus(es) 20 to the elongated member can be accomplished, so that the combination of apparatuses 20 and the elongated member hold the vertebrae in the corrected relative position. As already discussed, one apparatus as disclosed herein (e.g. apparatus 20) can be used with other types of orthopedic implants, apparatus, medicaments, osteogenic compounds or other items, and placement of such items can be done prior to, during, or after placement of the above-disclosed apparatus, as deemed appropriate by the surgeon.

It is to be understood that aspects or features described with respect to one embodiment may be used in or with respect to other embodiments. For example, an open or U-shaped channel (as in channel 34) may be incorporated with slotted wings such as wings 222 and 224. Similarly, in certain embodiments of a connecting apparatus, the wings may be different from each other. Thus, a connecting apparatus may have a central portion (e.g. central portion 22) with two wings, one of which is like wing 24 and one of which is like wing 224. Further, as noted above a variety of locking members and fixation elements may be used, depending on the configuration of the connecting apparatus and/or the needs or desires of the surgeon.

Referring now generally to FIGS 15-17, there is shown an embodiment of an elongated member 400 generally in the shape of a T that can be used with the apparatuses disclosed herein or with other types of orthopedic apparatus. Elongated member 400 includes a stem 402 and a cross-piece 404. Stem 402 is substantially straight (i.e. linear) with a longitudinal axis L and has a substantially flat surface 406, opposing curved surface 408, and side flat surfaces 410 in the illustrated embodiment. In one particular embodiment, surface 408 forms part of a cylinder, and a cross-section of stem 402 that is perpendicular to axis L is generally U-shaped. An end surface 412 of stem 402 is likewise generally U-shaped in the depicted embodiment. Similarly, the illustrated embodiment of cross-piece 404 is substantially straight (i.e. linear) with a longitudinal axis M and two branches 405a and 405b, and has a substantially flat surface 414, opposing curved surface 416, and side flat surfaces 418 in the illustrated embodiment. Surface 416 may form part of a cylinder, and a cross-section of cross-piece 404 perpendicular to axis M is generally U-shaped. End surfaces 420 of cross-piece 404 is likewise generally U-shaped in the depicted embodiment. Axes L and M are substantially perpendicular in this embodiment, although it will be seen that there may be a non-right angle between them through manufacturing of such an angle and/or by bending elongated member 400. In one particular embodiment, flat surfaces 414 and 406 are substantially coplanar, and stem 402 and cross-piece 404 are integral. Stem 402 is longer than cross-piece 404 in certain embodiments, but may also be approximately the same length or may be somewhat shorter. The illustrated embodiment of elongated member 400 shows stem 402 substantially centered with respect to cross-piece 404, that is, the lengths of branches 405a and 405b on either side of stem 402 are substantially the same.

Elongated member 400 may be used with any of the embodiments of apparatus 20, 120, 220, 320 described above, as well as other apparatuses. Stem 402 may be placed substantially along a portion of the length of the spinal column, in spinal applications, and in such cases cross-piece 404 may be connected at one or more points to a vertebra. Any one or all of stem 402 and branches 405a and 405b may be used with any of the apparatuses 20, 120, 220 and/or 320 disclosed herein. In one particular embodiment, in which stem 402 is substantially U-shaped and channel 34 of apparatus 20 has a similar shape, stem 402 may be inserted into channel 34 so that flat surface 406 of stem 402 generally faces threads 360 of apparatus 20. In that case, lock member 30 can contact surface 406 of stem 402. In another embodiment in which stem 402 is substantially U-shaped and channel 134 of apparatus 120 has an upper surface 133, surface 406 of stem 402 may be adjacent or facing surface 133 of apparatus 120. In that case, elongated member 400 may not be able to rotate with respect to channel 134 due to the proximity of surface 406 of elongated member 400 and surface 133 of apparatus 120.

While the disclosure has been illustrated and described in detail in the drawings and foregoing description, the same is to be considered as illustrative and not restrictive in character. It should be understood that only embodiments have been shown and described and that all changes and modifications that come within the scope of the claims are desired to be protected.

## Claims

1. An orthopedic medical apparatus comprising:
a connector (20) having a central portion (22), a first wing member (24) and a second wing member (26), said central portion (22) having a channel (34) for receiving at least part of an elongated member (R,400), said wing members (24,26) extending from said central portion (22) laterally with respect to said channel (34);
a first anchor member connected to said first wing member (24), said first anchor member adapted for anchoring to bone tissue; and
a second anchor member connected to said second wing member (26), said second anchor member adapted for anchoring to bone tissue;
**characterised in that** each wing member (24,26) has a lower surface (44) that is substantially cylindrical, upper side surfaces (46) that are substantially planar, and a top surface (48).

2. The apparatus of claim 1, wherein at least one of said wing members (24,26) is a solid rod, wherein optionally both of said wing members (24,26) are solid rods.

3. The apparatus of claim 1 or 2, wherein said channel (34) of said central portion (22) is open to the back of said central portion (22).

4. The apparatus of claim 1 or 2, wherein said channel (34) of said central portion (22) is closed to the back of said central portion (22) wherein optionally said central portion (22) includes a substantially flat surface adjoining a portion of said channel (34), wherein optionally said substantially flat surface adjoins a top portion of said channel; or

5. The apparatus of any preceding claim, wherein said wing members (24,26) each have a longitudinal axis, and said longitudinal axes are substantially collinear.

6. The apparatus of any of claims 1 to 4, wherein said wing members (24,26) each have a longitudinal axis, and said longitudinal axes are substantially coplanar.

7. The apparatus of any of claims 1 to 4, wherein said wing members (24,26) each have a longitudinal axis, said longitudinal axes including an angle between them, said angle being greater than 150 degrees, wherein optionally said angle is approximately 166 degrees.

8. The apparatus of any preceding claim, wherein said central portion (22) includes an arch below said channel (34).

9. The apparatus of any preceding claim, wherein said wing members (24,26) are integrally formed with said central portion (22).

10. The apparatus of claim 1, further comprising an elongated member (R,400) at least partially within said channel (34).

11. The apparatus of claim 10, wherein said elongated member (R,400) is at least partially a rod member.

12. The apparatus of claim 10, wherein said elongated member (R,400) is substantially T-shaped.

13. The apparatus of claim 12, wherein said elongated member (400) has a stem (402) and a cross-piece (404), and at least one of said stem (402) and said cross-piece (404) includes a substantially flat surface (406).

14. The apparatus of claim 12, wherein said elongated member (400) has a stem (402) and a cross-piece (404), and at least one of said stem (402) and said cross-piece (404) has a cross-section that is substantially U-shaped.

## Patentansprüche

1. Orthopädisch-medizinische Vorrichtung, die folgendes umfasst:
einen Verbinder (20) mit einem zentralen Teilstück (22), einem ersten Flügelelement (24) und einem zweiten Flügelelement (26), wobei das zentrale Teilstück (22) einen Kanal (34) zur Aufnahme zumindest eines Teils eines elongierten Elements (R, 400) aufweist, wobei sich die genannten Flügelelemente (24, 26) von dem genannten zentralen Teilstück (22) im Verhältnis zu dem genannten Kanal (34) lateral erstrecken;
ein erstes Ankerelement, das mit dem genannten ersten Flügelelement (24) verbunden ist, wobei das genannte erste Ankerelement eine Verankerung mit Knochengewebe vorsehen kann; und
ein zweites Ankerelement, das mit dem genannten zweiten Flügelelement (26) verbunden ist, wobei das genannte zweite Ankerelement eine Verankerung mit Knochengewebe vorsehen kann;
**dadurch gekennzeichnet, dass** jedes Flügelelement (24, 26) eine untere Oberfläche (44) aufweist, die im Wesentlichen zylindrisch ist, mit oberen Seitenoberflächen (46), die im Wesentlichen planar sind, und mit einer oberen Oberfläche (48).

2. Vorrichtung nach Anspruch 1, wobei zumindest eines der genannten Flügelelemente (24, 26) eine Massivstange ist, wobei optional die beiden genannten Flügelelemente (24, 26) Massivstangen sind.

3. Vorrichtung nach Anspruch 1 oder 2, wobei der genannte Kanal (34) des genannten zentralen Teilstücks (22) zu der Rückseite des genannten zentralen Teilstücks (22) offen ist.

4. Vorrichtung nach Anspruch 1 oder 2, wobei der genannte Kanal (34) des genannten zentralen Teilstücks zu der Rückseite des genannten zentralen Teilstücks (22) geschlossen ist, wobei das genannte zentrale Teilstück (22) optional eine im Wesentlichen flache Oberfläche aufweist, welche an ein Teilstück des genannten Kanals (34) angrenzt, wobei optional die genannte im Wesentlichen flache Oberfläche an ein oberes Teilstücke des genannten Kanals angrenzt.

5. Vorrichtung nach einem der vorstehenden Ansprüche, wobei die genannten Flügelelemente (24, 26) jeweils eine Längsachse aufweisen, und wobei die genannten Längsachsen im Wesentlichen kollinear sind.

6. Vorrichtung nach einem der Ansprüche 1 bis 4, wobei die genannten Flügelelemente (24, 26) jeweils eine Längsachse aufweisen, und wobei die genannten Längsachsen im Wesentlichen koplanar sind.

7. Vorrichtung nach einem der Ansprüche 1 bis 4, wobei die genannten Flügelelemente (24, 26) jeweils eine Längsachse aufweisen, und wobei die genannten Längsachsen einen Winkel zwischen den Achsen aufweisen, wobei der genannte Winkel größer ist als 150 Grad, wobei der genannte Winkel optional ungefähr 166 Grad ist.

8. Vorrichtung nach einem der vorstehenden Ansprüche, wobei das genannte zentrale Teilstück (22) eine Wölbung unter dem genannten Kanal (34) aufweist.

9. Vorrichtung nach einem der vorstehenden Ansprüche, wobei die genannten Flügelelemente (24, 26) integral mit dem genannten zentralen Teilstück (22) ausgebildet sind.

10. Vorrichtung nach Anspruch 1, wobei diese ferner ein elongiertes Element (R, 400) zumindest teilweise innerhalb des genannten Kanals (34) umfasst.

11. Vorrichtung nach Anspruch 10, wobei das genannte elongierte Element (R, 400) zumindest teilweise ein Stangenelement ist.

12. Vorrichtung nach Anspruch 10, wobei das genannte elongierte Element (R, 400) im Wesentlichen T-förmig ist.

13. Vorrichtung nach Anspruch 12, wobei das genannte elongierte Element (400) einen Schaft (402) und ein Kreuzstück (404) aufweist, und wobei zumindest der genannte Schaft (402) oder das genannte Kreuzstück (404) eine im Wesentlichen flache Oberfläche (406) aufweist.

14. Vorrichtung nach Anspruch 12, wobei das genannte elongierte Element (400) einen Schaft (402) und ein Kreuzstück (404) aufweist, und wobei zumindest der genannte Schaft (402) oder das genannte Kreuzstück (404) einen Querschnitt aufweist, der im Wesentlichen U-förmig ist.

## Revendications

1. Appareil médical orthopédique pouvant être implanté, comprenant :
un raccord (20) ayant une partie centrale (22), un premier élément formant ailette (24) et un second élément formant ailette (26), ladite partie centrale (22) ayant un canal (34) pour recevoir au moins une partie d'un élément allongé (R, 400), lesdits éléments formant ailettes (24, 26) s'étendant à partir de ladite partie centrale (22) latéralement par rapport audit canal (34) ;
un premier élément d'ancrage relié audit premier élément formant ailette (24), ledit premier élément d'ancrage étant conçu pour s'ancrer à un tissu osseux ; et
un second élément d'ancrage relié audit second élément formant ailette (26), ledit second élément d'ancrage étant conçu pour s'ancrer à un tissu osseux ;
**caractérisé en ce que** chaque élément formant ailette (24, 26) a une surface inférieure (44) qui est sensiblement cylindrique, des surfaces latérales supérieures (46) qui sont sensiblement planes, et une surface supérieure (48).

2. Appareil selon la revendication 1, dans lequel au moins l'un desdits éléments formant ailettes (24, 26) est une tige pleine, dans lequel éventuellement lesdits deux éléments formant ailettes (24, 26) sont des tiges pleines.

3. Appareil selon la revendication 1 ou 2, dans lequel ledit canal (34) de ladite partie centrale (22) est ouvert à l'arrière de ladite partie centrale (22).

4. Appareil selon la revendication 1 ou 2, dans lequel ledit canal (34) de ladite partie centrale (22) est fermé à l'arrière de ladite partie centrale (22), dans lequel éventuellement ladite partie centrale (22) comprend une surface sensiblement plane adjacente à une partie dudit canal (34), dans lequel éventuellement ladite surface sensiblement plane est adjacente à une partie supérieure dudit canal.

5. Appareil selon l'une quelconque des revendications précédentes, dans lequel lesdits éléments formant ailettes (24, 26) ont chacun un axe longitudinal, et lesdits axes longitudinaux sont sensiblement colinéaires.

6. Appareil selon l'une quelconque des revendications 1 à 4, dans lequel lesdits éléments formant ailettes (24, 26) ont chacun un axe longitudinal, et lesdits axes longitudinaux sont sensiblement coplanaires.

7. Appareil selon l'une quelconque des revendications 1 à 4, dans lequel lesdits éléments formant ailettes (24, 26) ont chacun un axe longitudinal, lesdits axes longitudinaux formant un angle entre eux, ledit angle étant supérieur à 150 degrés, dans lequel éventuellement ledit angle est d'environ 166 degrés.

8. Appareil selon l'une quelconque des revendications précédentes, dans lequel ladite partie centrale (22) comprend un arc au-dessous dudit canal (34).

9. Appareil selon l'une quelconque des revendications précédentes, dans lequel lesdits éléments formant ailettes (24, 26) font partie intégrante de ladite partie centrale (22).

10. Appareil selon la revendication 1, comprenant en outre un élément allongé (R, 400) au moins partiellement à l'intérieur dudit canal (34).

11. Appareil selon la revendication 10, dans lequel ledit élément allongé (R, 400) est au moins partiellement un élément formant tige.

12. Appareil selon la revendication 10, dans lequel ledit élément allongé (R, 400) est sensiblement en forme de T.

13. Appareil selon la revendication 12, dans lequel ledit élément allongé (400) a une tige (402) et une pièce transversale (404), et au moins ladite tige (402) et/ou ladite pièce transversale (404) comprennent une surface sensiblement plane (406).

14. Appareil selon la revendication 12, dans lequel ledit élément allongé (400) a une tige (402) et une pièce transversale (404), et au moins ladite tige (402) et/ou ladite pièce transversale (404) ont une section transversale qui est sensiblement en forme de U.
